(19) Europäisches Patentamt / European Patent Office / Office européen des brevets

(11) **EP 4 640 213 A1**

(12) **EUROPEAN PATENT APPLICATION**
published in accordance with Art. 153(4) EPC

(43) Date of publication:
29.10.2025 Bulletin 2025/44

(21) Application number: 23907470.1

(22) Date of filing: 27.11.2023

(51) International Patent Classification (IPC):
A61K 9/20 (2006.01)      A61K 31/4184 (2006.01)
A61K 31/7034 (2006.01)   A61K 45/06 (2006.01)
A61P 3/10 (2006.01)      A61P 9/12 (2006.01)
A61P 9/04 (2006.01)

(52) Cooperative Patent Classification (CPC):
A61K 9/20; A61K 31/351; A61K 31/4184;
A61K 31/7034; A61K 45/06; A61P 3/10;
A61P 9/04; A61P 9/12

(86) International application number:
PCT/KR2023/019185

(87) International publication number:
WO 2024/136184 (27.06.2024 Gazette 2024/26)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 20.12.2022 KR 20220179717

(71) Applicant: THPharm Corp.
Cheongju-si, Chungcheongbuk-do 28160 (KR)

(72) Inventors:
• HAN, Tae Hee
  Cheongju-si, Chungcheongbuk-do 28165 (KR)
• YOO, Yung Geun
  Seoul 06256 (KR)

(74) Representative: Cabinet Becker et Associés
25, rue Louis le Grand
75002 Paris (FR)

Remarks:
The complete document including Reference Table(s) and the Sequence Listing(s) can be downloaded from the EPO website

(54) **ORAL PHARMACEUTICAL PREPARATION COMPRISING SODIUM-GLUCOSE COTRANSPORTER-2 INHIBITOR AND ANGIOTENSIN II RECEPTOR BLOCKER**

(57) The present invention relates to an oral pharmaceutical preparation comprising a sodium-glucose cotransporter-2 inhibitor and an angiotensin II receptor blocker, which enables the sodium-glucose cotransporter-2 inhibitor and the angiotensin II receptor blocker, which have conventionally been taken separately, to be manufactured into a single combination that can be orally administered, and can be implemented in a relatively small size, thereby improving convenience of administration and patient compliance, and since there are no drug-drug interaction issues, the present invention can be effectively utilized in patients who need to take the medication for a long period of time, such as type 2 diabetes, hypertension, hypertension with diabetes, and heart failure.

EP 4 640 213 A1

[FIG. 1A]

24h Treatment

[FIG. 1B]

48h Treatment

# Description

## [Technical Field]

The present disclosure relates to an oral pharmaceutical preparation containing a sodium glucose cotransporter-2 inhibitor and an angiotensin II receptor blocker.

The present disclosure was made with the support of the National Research Foundation of "Local Government-University Cooperation-Based Regional Innovation Project (Chungbuk Regional Innovation Platform, Project Unique Number 1345370811, Project Number 2021RIS-001)" with the Ministry of Education's funding in 2023.

This research was supported by "Regional Innovation Strategy (RIS)" through the National Research Foundation of Korea(NRF) funded by the Ministry of Education(MOE)(2021RIS-001).

## [Background Art]

Hypertensive vascular disease as a major cause of death is a multifactorial disease caused by the complex interaction between various chronic diseases and other risks such as smoking, obesity, etc. In particular, the prevalence of hypertension with diabetes in Korea was reported to be approximately 26% (Korean Society of Hypertension, 2020 Hypertension Fact Sheet), and the number of patients with hypertension with diabetes is continuously increasing. The incidence of hypertension is approximately twice higher in diabetic patients than non-diabetic people, and it is known that hypertension may develop or deteriorate due to microvascular and macrovascular complications caused by diabetes. In addition, people with hypertension are known to have a high risk of suffering from diabetes, and it has been reported that diabetic patients have a mortality rate from cardiovascular disease that is 2 to 4 times higher than non-diabetic people.

Therefore, patients with hypertension with diabetes are likely to show different pathophysiology or drug treatment responses from patients with hypertension without diabetes, and conventional drug treatment methods may fail to control blood pressure or show poor results. In addition, the patients with hypertension with diabetes have to take many drugs in large doses, and thus often fail to achieve a desired therapeutic effect due to low medication convenience and low medication compliance. Therefore, there is a need for the development of combination therapies and combination preparations suitable for the treatment of patients with hypertension with diabetes.

A sodium-glucose cotransporter-2 (SGLT-2) inhibitor serves to inhibit the reabsorption of glucose from the blood to lower blood sugar levels and inhibit the secretion of inflammatory cytokines, and thus has been used to treat type 2 diabetes and cardiovascular diseases such as heart failure, etc. SGLT-2 inhibitors known to have been developed so far include Dapagliflozin, Empagliflozin, ipragliflozin, Canagliflozin, luceogliflozin, tofogliflozin, and the like.

Unlike an angiotensin converting enzyme inhibitor, an angiotensin II receptor blocker (ARB) primarily affects the smooth muscles and the adrenal glands and selectively blocks the binding of angiotensin II to angiotensin II receptor type 1 (AT1R) to lower blood pressure and thus is used as an antihypertensive. ARBs known to have been developed so far include Valsartan, candesartan, irbesartan, Telmisartan, eprosartan, olmesartan, and the like.

Meanwhile, among the ARBs, Telmisartan has a very large difference in solubility by pH of 100 times or more, and particularly, has low solubility at the pH of an absorption site in the body. In addition, when prepared as a combination with an SGLT-2 inhibitor such as Dapagliflozin, the low solubility of Telmisartan slows down the dissolution rate of the two ingredients due to interference with a sink condition in a dissolution solution. In addition, Telmisartan has a tendency to absorb moisture, which may affect the stability of Dapagliflozin, and Dapagliflozin has a low melting point and low moisture stability, which has a difficulty in developing a combination of Telmisartan and the SGLT-2 inhibitor. Therefore, since Telmisartan and the SGLT-2 inhibitor need to currently be taken separately, there have been problems with low convenience of administration and drug compliance in patients with type 2 diabetes, hypertension, hypertension with diabetes, or heart failure, who need to take both the drugs.

Accordingly, the present inventors conducted research to develop an oral SGLT-2 inhibitor and ARB combination capable of maintaining a stable formulation without drug-drug interaction issues, and then completed the present disclosure.

## [Disclosure]

## [Technical Problem]

One object of the present disclosure is to provide an oral pharmaceutical preparation including: Telmisartan or a pharmaceutically acceptable salt thereof as a first pharmaceutical ingredient; a sodium-glucose cotransporter-2 (SGLT-2) inhibitor or a pharmaceutically acceptable salt thereof as a second pharmaceutical ingredient; and an excipient.

Another object of the present disclosure is to provide a pharmaceutical composition for preventing or treating type 2 diabetes, hypertension, hypertension with diabetes, or heart failure, including the oral pharmaceutical preparation.

Yet another object of the present disclosure is to provide a method for preventing or treating type 2 diabetes, hypertension, hypertension with diabetes, or heart failure, including administering the oral pharmaceutical preparation to a subject.

**[Technical Solution]**

One aspect of the present disclosure provides an oral pharmaceutical preparation including: Telmisartan or a pharmaceutically acceptable salt thereof as a first pharmaceutical ingredient; a sodium-glucose cotransporter-2 (SGLT-2) inhibitor or a pharmaceutically acceptable salt thereof as a second pharmaceutical ingredient; and an excipient.

According to an embodiment of the present disclosure, the SGLT-2 inhibitor may be Dapagliflozin or Empagliflozin.

According to an embodiment of the present disclosure, the weight ratio of the first pharmaceutical ingredient and the second pharmaceutical ingredient may be 4 : 1 to 8 : 1.

According to an embodiment of the present disclosure, the weight ratio of the entire pharmaceutical ingredient and the excipient may be 1 : 2 to 1 : 5.

According to an embodiment of the present disclosure, the preparation may be a tablet.

According to an embodiment of the present disclosure, the tablet may include 40 to 80 mg of the first pharmaceutical ingredient; and 10 mg of the second pharmaceutical ingredient.

According to an embodiment of the present disclosure, when the tablet is subjected to a dissolution test at a paddle rotation rate of 50 rpm in a pH 1.2 hydrochloric acid solution according to a dissolution test method 2 of the Korean Pharmacopoeia, the dissolution rate of the first pharmaceutical ingredient may be 40% or more for 30 minutes and 85% or more for 90 minutes, and the dissolution rate of the second pharmaceutical ingredient may be 60% or more for 5 minutes and 95% or more for 30 minutes.

Another aspect of the present disclosure provides a pharmaceutical composition for preventing or treating type 2 diabetes, hypertension, hypertension with diabetes, or heart failure, including the oral pharmaceutical preparation.

Yet another aspect of the present disclosure provides a method for preventing or treating type 2 diabetes, hypertension, hypertension with diabetes, or heart failure, including administering the oral pharmaceutical preparation to a subject.

**[Advantageous Effects]**

According to the present disclosure, the oral pharmaceutical preparation including the sodium-glucose cotransporter-2 inhibitor and the angiotensin II receptor blocker enables the sodium-glucose cotransporter-2 inhibitor and the angiotensin II receptor blocker, which have conventionally been taken separately, to be manufactured into a single combination that can be orally administered, and can be implemented in a relatively small size, thereby improving convenience of administration and patient compliance, and since there are no drug-drug interaction issues, the present disclosure can be effectively utilized in patients who need to take the medication for a long period of time, such as type 2 diabetes, hypertension, hypertension with diabetes, and heart failure.

**[Description of Drawings]**

FIG. 1A is a graph showing results of measuring cell survival rates after 24 hours of treatment with Telmisartan or olmesartan in A549 cells.

FIG. 1B is a graph showing results of measuring cell survival rates after 48 hours of treatment with Telmisartan or olmesartan in A549 cells.

FIG. 2A is a graph showing results of measuring cell survival rates after 24 hours of treatment with Telmisartan or olmesartan in MDCK cells.

FIG. 2B is a graph showing results of measuring cell survival rates after 48 hours of treatment with Telmisartan or olmesartan in MDCK cells.

FIG. 3 is a graph showing results of evaluating the inhibitory activity of an angiotensin converting enzyme (ACE) after treatment with Telmisartan or olmesartan in a cardiac muscle cell line H9c2 with inflammation induced by lipopolysaccharide (LPS).

FIG. 4 is a graph showing results of evaluating the inhibitory activity on ACE mRNA expression after treatment with Telmisartan or olmesartan in the cardiac muscle cell line H9c2 induced with inflammation by LPS.

FIG. 5 is a diagram showing results of evaluating the inhibitory activity on ACE mRNA expression after treatment with various sodium glucose cotransporter-2 inhibitors and/or angiotensin II receptor blockers in the cardiac muscle cell line H9c2 induced with inflammation by LPS.

FIG. 6 is a graph showing results of evaluating the inhibitory activity on ACE mRNA expression after treatment with various sodium glucose cotransporter-2 inhibitors and/or angiotensin II receptor blockers in the cardiac muscle cell line H9c2 induced with inflammation by LPS.

FIG. 7 is a graph showing results of evaluating the inhibitory activity on ACE protein expression after treatment with various sodium glucose cotransporter-2 inhibitors and/or angiotensin II receptor blockers in the cardiac muscle cell line H9c2 induced with inflammation by LPS.

FIG. 8 is a graph showing a dissolution pattern of Telmisartan in a mixed bilayer tablet of Telmisartan and Dapagliflozin according to an embodiment of the present disclosure.

FIG. 9 is a graph showing a dissolution pattern of Dapagliflozin in a mixed bilayer tablet of Telmisartan and Dapagliflozin according to an embodiment of the present disclosure.

FIG. 10 is a graph showing pharmacokinetics of Dapagliflozin according to single oral administration of a combined bilayer tablet of Dapagliflozin and Telmisartan according to an embodiment of the present disclosure.

FIG. 11 is a graph showing pharmacokinetics of Telmisartan according to single oral administration of a combined bilayer tablet of Dapagliflozin and Telmisartan according to an embodiment of the present disclosure.

FIG. 12A is a diagram showing results of evaluating body weight loss and heart weight loss effects of Dapagliflozin and Telmisartan combination administration groups of 1/8 mg/kg/day (low-dose group, G2), 3/24 mg/kg/day (medium-dose group, G3), and 9/72 mg/kg/day (high-dose group, G4), each single administration group of Dapagliflozin 9 mg/kg/day (G5) and Telmisartan 72 mg/kg/day (G6), respectively, and a control group (0.5% MC aqueous solution, G1), in male rats.

FIG. 12B is a diagram showing results of evaluating body weight loss and heart weight loss effects of Dapagliflozin and Telmisartan combination administration groups of 1/8 mg/kg/day (low-dose group, G2), 3/24 mg/kg/day (medium-dose group, G3), and 9/72 mg/kg/day (high-dose group, G4), each single administration group of Dapagliflozin 9 mg/kg/day (G5) and Telmisartan 72 mg/kg/day (G6), respectively, and a control group (0.5% MC aqueous solution, G1), in female rats.

**[Best Mode]**

One aspect of the present disclosure provides an oral pharmaceutical preparation including: Telmisartan or a pharmaceutically acceptable salt thereof as a first pharmaceutical ingredient; a sodium-glucose cotransporter-2 (SGLT-2) inhibitor or a pharmaceutically acceptable salt thereof as a second pharmaceutical ingredient; and an excipient.

In the present disclosure, in order to enable sodium-glucose cotransporter-2 (SGLT-2) and an angiotensin II receptor blocker (ARB), which have conventionally been taken separately, to be manufactured into a single combination that may be taken orally, the angiotensin converting enzyme inhibitory activity of various SGLT-2s and ARBs was evaluated, and a drug-drug compatibility test and a preparation stability analysis were performed to derive an optimal combination of SGLT-2 and ARB. In addition, the toxicity, dissolution characteristics, and pharmacokinetics of the derived combination were evaluated, and as a result, it was confirmed that an oral pharmaceutical formulation according to an embodiment of the present disclosure may be effectively applied to the prevention or treatment of type 2 diabetes, hypertension, hypertension with diabetes, or heart failure.

Specifically, in the present disclosure, it was confirmed that Telmisartan has a superior angiotensin converting enzyme inhibitory effect to olmesartan, and Telmisartan has superior combination compatibility and preparation stability with SGLT-2 inhibitors, particularly Dapagliflozin and Empagliflozin, compared to olmesartan and Valsartan among ARBs. Accordingly, an oral formulation with excellent preparation stability, improved dissolution rate, and improved combination compatibility with pharmaceutical additives was developed.

The SGLT-2 inhibitor included in the oral pharmaceutical preparation of the present disclosure may be any one substance selected from the group consisting of Dapagliflozin, Empagliflozin, ipragliflozin, Canagliflozin, luseogliflozin, and tofogliflozin, but preferably Dapagliflozin or Empagliflozin in terms of combination compatibility and preparation stability.

The oral pharmaceutical preparation of the present disclosure may be, for example, a bilayer tablet including a first layer including Telmisartan as a first pharmaceutical ingredient and a second layer including an SGLT-2 inhibitor as a second pharmaceutical ingredient, or may be an inner core tablet structure including an inner core layer including Telmisartan as a first pharmaceutical ingredient and an outer layer including an SGLT-2 inhibitor as a second pharmaceutical ingredient and surrounding the inner core layer, but is not limited thereto.

According to conventional reports, it was reported that Telmisartan has a problem of interfering with the dissolution of SGLT-2 inhibitors, particularly Dapagliflozin. Specifically, Telmisartan has particularly low solubility at pH of the duodenum or the small intestine, which is the absorption site in the body, and has a dissolution rate lowered due to interference with a sink condition in the dissolution solution when preparing a combination of the two ingredients. In addition, in the case of Dapagliflozin, which is an immediate-release preparation ingredient, the dissolution rate and the dissolution speed are slow, which affects the pharmacokinetics, and in the case of Telmisartan, there is a tendency of absorbing moisture, which affects the stability of Dapagliflozin. On the other hand, in the oral pharmaceutical preparation of the present disclosure, a problem of dissolution interference of Telmisartan has been solved. That is, the oral pharmaceutical preparation of the present disclosure includes Telmisartan as the first pharmaceutical ingredient and the SGLT-2 inhibitor as the second pharmaceutical ingredient, and since the first and second pharmaceutical ingredients have no interaction problems such as dissolution interference between each ingredient, only taking one tablet of the oral pharmaceutical preparation of the present disclosure once a day may exhibit the same effect as taking each tablet of a conventional Telmisartan single tablet and a conventional SGLT-2 inhibitor single tablet, once a day.

As used in the present disclosure, the term "oral pharmaceutical preparation" refers to a preparation made by molding or coating a pharmaceutical ingredient into a predetermined shape, and for example, the oral pharmaceutical preparation may be formulated as a dry syrup, granules, tablets (monolayer tablets, bilayer tablets, inner-core tablets, etc.), pellets, or capsules, but is not limited thereto. The oral pharmaceutical preparation may be a preparation in which wet granules are filled in the form of tablets, pellets, or capsules. The tablets, pellets and capsules may be commonly used in the art. The capsule may be a hard capsule or a soft capsule. When the oral pharmaceutical preparation is the capsule, the capsule may be prepared in a form including granules, tablets, or the like therein.

The dose of the oral pharmaceutical preparation according to an embodiment of the present disclosure may be, for example, within the range of about 0.001 mg/kg to about 100 mg/kg, about 0.01 mg/kg to about 10 mg/kg, or about 0.1 mg/kg to about 1 mg/kg for adults, may be administered once a day, multiple times a day, or once a week, once every two weeks, once every three weeks, or once every four weeks to once a year, and may be separately administered once or several times a day at regular intervals according to the judgment of a doctor or pharmacist.

The oral pharmaceutical preparation of the present disclosure may be prepared by mixing Telmisartan as the first pharmaceutical ingredient and the SGLT-2 inhibitor as the second pharmaceutical ingredient with the excipient. The mixing thereof may be performed simultaneously or sequentially, and may be performed using methods known in the art.

According to an embodiment of the present disclosure, the SGLT-2 inhibitor may be Dapagliflozin or Empagliflozin.

As used in the present disclosure, the term "Dapagliflozin" refers to (1S)-1,5-anhydro-1-C-{4-chloro-3-[(4-ethoxyphenyl)methyl]phenyl}-D-glucitol, and "Empagliflozin" refers to (1S)-1,5-anhydro-1-C-[4-chloro-3-[[4-[[(3S)-tetrahydro-3-furanyl]oxy]phenyl]methyl]phenyl]-D-glucitol. As used herein, the term "Telmisartan" refers to 4'-[(1,4'-dimethyl-2'-propyl[2,6'-bi-1H-benzimidazol]-1'-yl)methyl][1,1'-biphenyl]-2-carboxylic acid.

The Dapagliflozin, Empagliflozin or Telmisartan may be referred to including both active metabolites and prodrugs thereof. The "metabolite" is each active derivative that may be produced when Dapagliflozin, Empagliflozin or Telmisartan is metabolized, and the "prodrug" refers to a compound that is metabolized to Dapagliflozin, Empagliflozin or Telmisartan, or to the same metabolite(s) as Dapagliflozin, Empagliflozin or Telmisartan. In addition, Dapagliflozin, Empagliflozin or Telmisartan may include all pharmaceutically acceptable salts thereof, crystal forms thereof, hydrates, solvates, diastereomers or enantiomers.

For example, the term "Dapagliflozin" used in the present disclosure is used with the same meaning as "Dapagliflozin propanediol hydrate".

According to an embodiment of the present disclosure, the weight ratio of the first pharmaceutical ingredient and the second pharmaceutical ingredient may be 4 : 1 to 8:1.

When the first pharmaceutical ingredient is included in an amount of less than 4 weights based on 1 weight of the second pharmaceutical ingredient, an appropriate therapeutic effect may not be expected due to a low drug blood concentration and a low pharmaceutical effect resulting therefrom. In addition, when the first pharmaceutical ingredient is included in an amount of more than 8 weights based on 1 weight of the second pharmaceutical ingredient, there may be problems such as a high drug blood concentration and side effects resulting therefrom, expression of toxicity, and induction of hypoglycemia and hypotension due to excessive therapeutic effects, and an appropriate therapeutic effect may not be expected.

According to an embodiment of the present disclosure, the weight ratio of the entire pharmaceutical ingredient and the excipient may be 1 : 2 to 1 : 5.

When the excipient is included in an amount of less than 2 weights based on 1 weight of the entire pharmaceutical ingredient, a release pattern of each drug may vary by solubility interactions in the dissolution solution, and there is a possibility of a toxicity problem due to excessive release. On the other hand, if the excipient is included in an amount of more than 5 weights based on 1 weight of the entire pharmaceutical ingredient, the size of the oral preparation may become excessively large, which may cause inconvenience when taking the drug, there may be problems that drug compliance may decrease, and the release pattern may vary due to delays in the release of each drug caused by excessive use of the excipient, and the like.

According to an embodiment of the present disclosure, the preparation may be a tablet.

For the convenience of medication of patients, when the tablet according to an embodiment of the present disclosure is a bilayer tablet formulation, it is preferable that the first layer containing the first pharmaceutical ingredient is 500 mg or less and the second layer containing the second pharmaceutical ingredient is 300 mg or less, and when the tablet is an active coating formulation, it is preferable that the inner core layer containing the first pharmaceutical ingredient is 500 mg or less and the outer layer containing the second pharmaceutical ingredient is 50 mg or less.

When the oral pharmaceutical preparation of the present disclosure is a tablet, the tablet may further include one or more pharmaceutically acceptable additives selected from the group consisting of an excipient, specifically a diluent, a disintegrant, a lubricant, a binder, a stabilizer, and a coating agent.

The excipient, specifically the diluent, may be, for example, lactose or hydrates thereof, cellulose derivatives including microcrystalline cellulose, starch, gelled starch, sucrose, sugar alcohols including Ludipress® mannitol, sorbitol, and the like, inorganic salts including calcium phosphate, aluminum silicate, calcium sulfate, and the like, but is not limited thereto.

The disintegrant may be one or more ingredients selected from the group consisting of, for example, polyvinylpyrrolidone, croscarmellose sodium, sodium starch glycolate, corn starch, crospovidone, low-substituted hydroxypropyl cellulose, and gelatinized starch, but is not limited thereto.

The lubricant may be, for example, one or more ingredients selected from the group consisting of stearic acid, stearic acid metal salts (e.g., calcium stearate, magnesium stearate, etc.), talc, colloidal silica, sucrose fatty acid esters, hydrogenated vegetable oils, waxes, glyceryl fatty acid esters, and glycerol dibehenate, but is not limited thereto. In one specific example, the lubricant may be magnesium stearate.

The binder refers to an "external granule phase" binder which is added after completion of a wet granulation step. The granule external phase binder may be, for example, one or more ingredients selected from the group consisting of hydroxypropyl cellulose (HPC), copovidone (copolymer of vinylpyrrolidone with other vinyl derivatives), hydroxypropyl methylcellulose (HPMC), polyvinyl pyrrolidone (povidone), and macrogol, but is not limited thereto.

The stabilizer may be, for example, an antioxidant, an acidifying agent, or an alkalizing agent, but is not limited thereto.

The coating agent may be, for example, hypromellose, polyvinyl alcohol, ethyl cellulose, titanium oxide, polyethylene glycol, and Opadry®, but is not limited thereto. The coating agent may be included in an amount of 0.1 to 10 wt%, preferably 2 to 7 wt%, and most preferably about 5 wt% based on the total weight of the oral pharmaceutical preparation of the present disclosure, but is not limited thereto.

According to an embodiment of the present disclosure, the tablet may include 40 mg to 80 mg of the first pharmaceutical ingredient; and 10 mg of the second pharmaceutical ingredient.

When the first pharmaceutical ingredient is included in less than 40 mg and/or the second pharmaceutical ingredient is included in less than 10 mg, an appropriate therapeutic effect for diabetes and hypertension may not be expected due to a low drug blood concentration and a low pharmaceutical effect resulting therefrom. On the other hand, when the first pharmaceutical ingredient is included in more than 80 mg and/or the second pharmaceutical ingredient is included in more than 10 mg, there may be problems, such as a high drug blood concentration and side effects resulting therefrom, expression of toxicity, and induction of hypoglycemia and hypotension due to excessive therapeutic effects, and an appropriate therapeutic effect may not be expected. In an embodiment, the tablet may include 40 mg and 10 mg or 80 mg and 10 mg of the first pharmaceutical ingredient and the second pharmaceutical ingredient, respectively.

Meanwhile, 12.3 mg of the Dapagliflozin propanediol hydrate is equivalent to 10 mg when converted to Dapagliflozin as the pharmaceutical ingredient.

According to an embodiment of the present disclosure, when the tablet is subjected to a dissolution test at a paddle rotation rate of 50 rpm in a pH 1.2 hydrochloric acid solution according to a dissolution test method 2 of the Korean Pharmacopoeia, the dissolution rate of the first pharmaceutical ingredient may be 40% or more for 30 minutes and 85% or more for 90 minutes, and the dissolution rate of the second pharmaceutical ingredient may be 60% or more for 5 minutes and 95% or more within 30 minutes.

The tablet may have the dissolution rate of the first pharmaceutical ingredient of 40%, 45%, 50%, 55%, 60% or 70% for 30 minutes and 85%, 90%, 95 or 99% for 90 minutes, during the dissolution test. In addition, the dissolution rate of the second pharmaceutical ingredient may be 60%, 65% or 70% for 5 minutes, and 95% or 99% within 30 minutes.

The dissolution test may be performed using a paddle method according to the dissolution test method 2 of the Korean Pharmacopoeia. The dissolution test may be performed using a pH 1.2 simulated gastric fluid (SGF) or a 0.1 N hydrochloric acid solution. The dissolution test may be performed at 40 to 60 revolutions per minute (rpm), for example, 50 rpm. The dissolution test may be performed at about 30°C to about 40°C, about 32°C to about 40°C, about 34°C to about 40°C, about 36°C to about 40°C, or about 37°C.

In an embodiment, when the tablet is subjected to the dissolution test at the paddle rotation rate of 50 rpm in the pH 1.2 hydrochloric acid solution according to the dissolution test method 2 of the Korean Pharmacopoeia, the dissolution rate of Telmisartan as the first pharmaceutical ingredient may be 40% or more for 30 minutes and 85% or more for 90 minutes, and the dissolution rate of Dapagliflozin as the second pharmaceutical ingredient may be 60% or more for 5 minutes and 95% or more for 30 minutes.

Another aspect of the present disclosure provides a pharmaceutical composition for preventing or treating type 2

# EP 4 640 213 A1

diabetes, hypertension, hypertension with diabetes, or heart failure, including the oral pharmaceutical preparation.

The composition of the present disclosure may include a pharmaceutically acceptable carrier. The pharmaceutically acceptable carrier included in the composition of the present disclosure is generally used in preparation of drugs, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like, but is not limited thereto. The pharmaceutical composition of the present disclosure may further include a lubricant, a wetting agent, a sweetening agent, a flavoring agent, an emulsion, a suspension, a preservative, and the like, in addition to the ingredients. Suitable pharmaceutically acceptable carriers and preparations are described in detail in Remington: the science and practice of pharmacy 22nd edition (2013).

The pharmaceutical composition according to an embodiment of the present disclosure may be administered together with a substance exhibiting the pharmaceutical activity for one or more of type 2 diabetes, hypertension, hypertension with diabetes, and/or heart failure.

In addition, the pharmaceutical composition according to an embodiment of the present disclosure may be used alone or in combination with methods using surgery, hormone therapy, drug therapy, and/or biological response regulators, for the prevention or treatment of type 2 diabetes, hypertension, hypertension with diabetes, and/or heart failure.

The composition of the present disclosure may include various bases and/or additives necessary and appropriate for the preparation of formulations thereof, and without departing from the range of reducing the effects thereof, may be prepared by further including known compounds, such as nonionic surfactants, silicone polymers, extenders, fragrances, preservatives, disinfectants, oxidation stabilizers, organic solvents, ionic or nonionic thickeners, softeners, antioxidants, free radical destroyers, opacifiers, stabilizers, emollients, silicone, $\alpha$-hydroxy acid, anti-foaming agents, moisturizers, vitamins, insect repellents, flavorings, preservatives, surfactants, anti-inflammatory agents, substance P antagonists, fillers, polymers, propellants, alkalinizing or acidifying agents, or coloring agents.

A suitable dose of the composition of the present disclosure may be variously prescribed by factors, such as a formulation method, an administration method, age, weight, sex, and a pathological condition of a patient, diet, an administration time, an administration route, an excretion rate, and response susceptibility. The dose of the composition of the present disclosure may be 0.001 to 1000 mg/kg for adults.

The composition of the present disclosure may be orally administered.

The composition of the present disclosure may be administered in various formulations when administered orally, and may be administered in the form of tablets, pills, hard/soft capsules, liquids, suspensions, emulsifiers, syrups, granules, elixirs, troches, etc., and may further include various excipients, such as wetting agents, sweeteners, aromatics, preservatives, etc. Specifically, when the composition of the present disclosure is prepared in the form of oral administration formulations, the composition may further include suitable carriers, excipients and diluents commonly used in preparation thereof. The carriers, excipients and diluents may be used with, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and/or mineral oil, but are not limited thereto. In addition, the composition may be prepared by including diluents or excipients, such as fillers, extenders, binders, wetting agents, disintegrants, and surfactants commonly used in formulation, and may further include lubricants such as magnesium stearate or talc, in addition to the excipients.

Yet another aspect of the present disclosure provides a method for preventing or treating type 2 diabetes, hypertension, hypertension with diabetes, or heart failure, including administering the oral pharmaceutical preparation to a subject.

A telmisartan or a pharmaceutically acceptable salt thereof, and an SGLT-2 inhibitor or a pharmaceutically acceptable salt thereof, included in the oral pharmaceutical preparation of the present disclosure, may be administered orally in an amount effective for the treatment or prevention of a subject or patient, depending on the intended purpose. It should be understood that the dose for a specific subject or patient should be determined based on various related factors, such as the patient's weight, age, race, sex, health condition, diet, administration time, administration method, and severity of disease, and may be appropriately increased or decreased by a specialist. For example, a doctor may gradually increase the dose of the oral pharmaceutical preparation of the present disclosure at a level lower than that required to achieve a desired therapeutic effect until the desired effect is achieved, and may easily determine and prescribe the dose as needed.

## [Modes of the Invention]

Hereinafter, the present disclosure will be described in more detail with reference to Examples. However, these Examples are only illustrative the present disclosure, and the scope of the present disclosure is not limited to these Examples.

**Experimental Example 1. Screening of angiotensin II receptor blockers for use in combination for treatment of heart failure**

*1-1. Evaluation of cell survival rate*

To screen angiotensin II receptor blockers for use in combinations of angiotensin II receptor blockers (ARBs) and sodium-glucose cotransporter-2 (SGLT-2) inhibitors for the treatment of heart failure, cell survival rates were evaluated.

Specifically, A549 cells were obtained from the Korean Cell Line Bank (KCLB) and subcultured using a RPMI-1640 medium supplemented with 10% fetal bovine serum (FBS) and 1% Penicillin, and MDCK cells were obtained from ATCC and subcultured using a DMEM medium supplemented with 10% FBS. 200 $\mu$l each of the A549 cells and MDCK cells incubated in an incubator under 37°C and 5% $CO_2$ conditions were dispensed at $2 \times 10^4$ cells/well in a 96-well plate, and then a control group was treated with 100 $\mu$l of the medium, and experimental groups were treated with 1.25, 25, 50, and 100 $\mu$M of Telmisartan or olmesartan, respectively. After 24 and 48 hours, all of the wells were treated with 50 $\mu$l each of a 2 mg/ml MTT reagent and incubated in an incubator for 3 hours, and then each well was added with 500 $\mu$l of DMSO, wrapped in foil, stirred for 30 minutes at room temperature, and the absorbance was measured at 570 nm to obtain the cell survival rate.

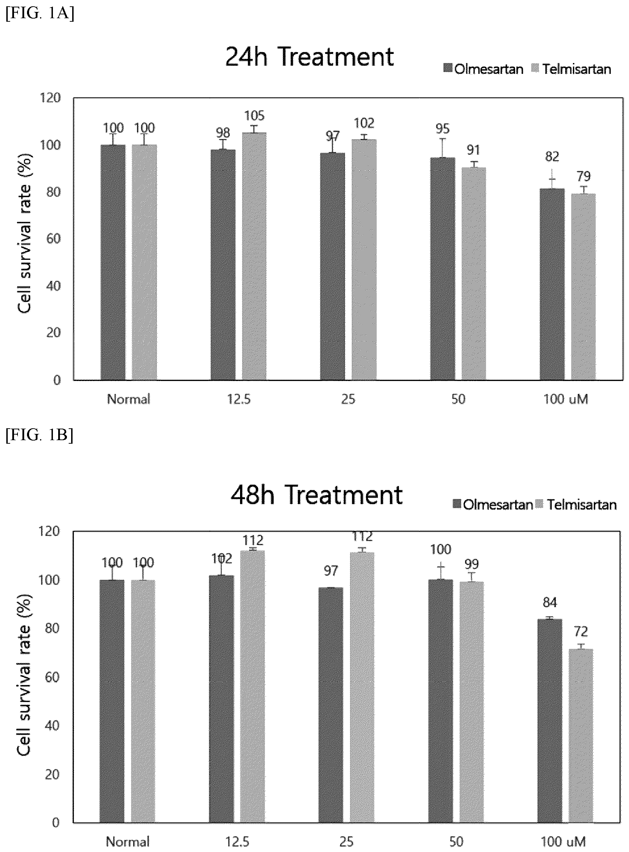

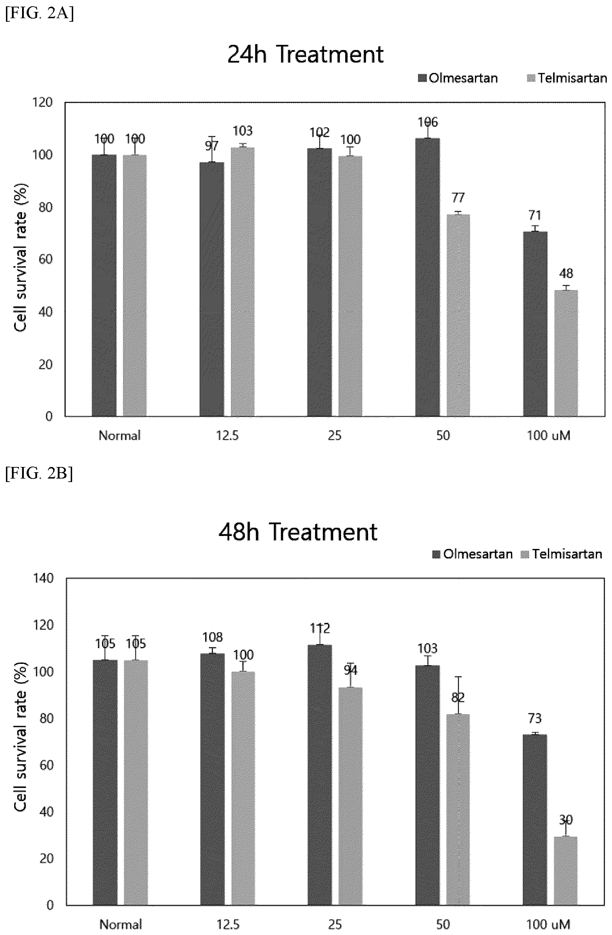

As a result, it was confirmed that both Telmisartan and olmesartan had no effect on cell survival rate up to a concentration of 25 $\mu$M (FIGS. 1A, 1B, 2A and 2B).

*1-2. Evaluation of angiotensin converting enzyme inhibitory activity*

To confirm a heart failure therapeutic effect of an angiotensin II receptor blocker (ARB), an angiotensin converting enzyme (ACE) inhibitory activity was evaluated.

Specifically, H9c2 cells as a human cardiac cell line were obtained and used from the Korea Cell Line Bank (KCLB 40071, Korea) and incubated and prepared in a DMEM medium supplemented with 10% FBS and 1% antibiotic-antimycotic (10 units/ml penicillin, 100 $\mu$g/ml streptomycin, 0.25 $\mu$g/ml amphotericin) under 37°C and 5% $CO_2$ conditions.

To confirm the effect on ACE activity, the prepared H9c2 cells were treated with 2.5 $\mu$g/ml of Lipopolysaccharide (LPS), which was 2.5 times more than the amount used in conventional inflammation-related experiments, to induce inflammation, thereby setting the optimized conditions for confirming ACE expression. LPS inflammation-induced H9c2 cells were treated with 10, 25, and 50 $\mu$M Telmisartan or olmesartan, respectively, and the H9c2 cells were harvested for ELISA assay. The harvested cells were treated with 1 ml of an RIPA buffer and stirred at 4°C for 30 minutes to extract proteins. Thereafter, the proteins were centrifuged at 4°C and 10,000 rpm for 20 minutes, and the supernatant was transferred to a sterilized tube and stored at - 80°C for use in the ELISA assay.

To perform the ELISA assay, 10,000 pg/ml, 5,000 pg/ml, 2,500 pg/ml, 1,250 pg/ml, 625 pg/ml, 312 pg/ml, and 156 pg/ml of Human ACE standard solutions and a standard dilution buffer were added by 0.1 ml to each well of an antibody-coated 96-well plate. Thereafter, 0.1 ml of ACE protein extracted from H9c2 cells was added to each well, and then the plate was sealed with a cover, and incubated at 37°C for 90 minutes. In the experiment, the ACE protein was treated so that 20 $\mu$g was added to 0.1 ml of a solution to be treated, and 0.08 ml of a sample dilution buffer was treated, so that the final dilution ratio was 1 : 5. After incubation, the cover was removed, the contents of the plate were removed, and then any residue was removed with a paper towel or other absorbent materials, while preventing the wells from drying completely. After washing, each well was treated with 0.1 ml of a biotinylated Anti-Human ACE antibody solution included in a Human ACE ELISA kit and incubated at 37°C for 60 minutes. After incubation, the plate was washed three times with 0.01 M PBS, and the residual PBS solution was removed with a paper towel or other absorbent materials. After washing, each well was added with 0.1 ml of the ABC solution included in the Human ACE ELISA kit, incubated at 37°C for 30 minutes, and then the plate was washed five additional times with 0.01 M PBS. Thereafter, each well was added with 90 $\mu$l of the prepared TMB solution, incubated for 20 to 25 minutes in a dark room at 37°C, and then treated with each 0.1 ml of the prepared TMB stop solution to stop an ACE ELISA reaction, and the OD absorbance at 450 nm was read in a microplate reader within 30 minutes, and the relative OD450 was derived using the following Equation.

$$\text{The relative OD450} = (\text{the OD450 of each well}) - (\text{the OD450 of Zero well})$$

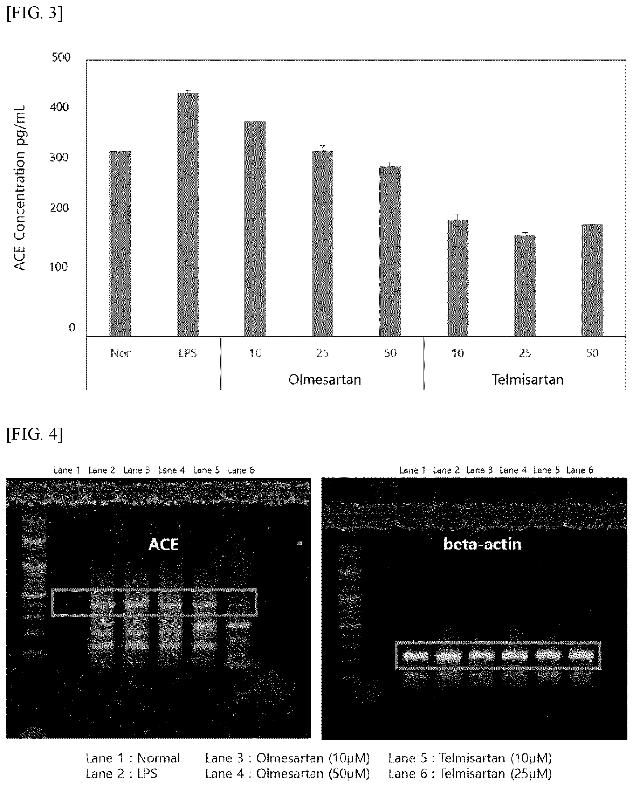

As a result, it was confirmed that an ACE expression inhibition effect of Telmisartan was superior to that of olmesartan at all treatment concentrations, and the ACE expression inhibition effect was the best in a 25 $\mu$M Telmisartan treatment group (FIG. 3).

*1-3. Evaluation of angiotensin converting enzyme gene expression*

To confirm a heart failure therapeutic effect of an angiotensin II receptor blocker (ARB), it was confirmed whether the mRNA expression of an angiotensin converting enzyme (ACE) was inhibited.

Specifically, LPS inflammation-induced H9c2 cells in Experimental Example 1-2 were treated with 10 or 25 $\mu$M Telmisartan and 10 or 50 $\mu$M olmesartan, and then treated with 200 $\mu$l of chloroform together with 1 ml of TRIZol (Sigma, USA), gently mixed, and separated for 10 minutes, and then centrifuged at 4°C and 10,000 rpm for 10 minutes. After centrifugation, only the supernatant from the tube was taken and transferred to a new sterile tube, and the supernatant was treated with 500 $\mu$l of isopropanol to precipitate RNA. The precipitated RNA was centrifuged at 4°C, 10,000 rpm for 10 minutes, and then the supernatant except for the pellets in the tube was fully removed, and the RNA pellets were treated with 500 $\mu$l of 75% ethyl alcohol and thoroughly washed. The washed pellets were centrifuged at 4°C, 10,000 rpm for 5 minutes, and then the supernatant except the pellets was fully removed. The tube containing the pellets from which the supernatant had been cleanly removed was treated with diethyl pyrocarbonate (DEPC)-water and stored at - 80°C for use in an RT-PCR experiment.

RT-PCR was performed by synthesizing cDNA from mRNA using an RT-PCR kit containing DNA polymerase, buffer, dNTP, and tracking dye, and then using ACE primers in Table 1 under the conditions in Table 2 for 35 cycles. The PCR products were electrophoresed on a 15% agarose gel, identified using a UV transilluminator, and expressed as normalized to GAPDH for quantitative comparison.

[Table 1]

| Primer name | | Nucleic acid sequence (5'→3') | SEQ ID NO: |
|---|---|---|---|
| ACE (510 bp) | forward | CAACGCCCTGCTAAGCAAC | SEQ ID NO: 1 |
| | reverse | ACTGGTGACATCGAGGTTGG | SEQ ID NO: 2 |
| GAPDH (274 bp) | forward | CAGTCAACGGATTTGGTCGT | SEQ ID NO: 3 |
| | reverse | TTGATTTTGCAGGGATCTCG | SEQ ID NO: 4 |

[Table 2]

| Classification | Temperature | Time |
|---|---|---|
| Denaturation | 94°C | 30 sec |
| Annealing | 55°C to 62°C | 30 sec |
| Elongation | 72°C | 60 sec |

As a result, it was confirmed that an mRNA expression inhibition effect of Telmisartan was superior to that of olmesartan at all treatment concentrations, and the mRNA expression was mostly inhibited in a 25 $\mu$M Telmisartan treatment group (FIG. 4).

Through these results, it was confirmed that Telmisartan was more effective than olmesartan, as an angiotensin II receptor blocker for use in a combination of the angiotensin II receptor blocker and the sodium glucose cotransporter-2 inhibitor for the treatment of heart failure.

**Experimental Example 2. Confirmation of angiotensin converting enzyme inhibitory activity according to mRNA expression inhibition of ARB and SGLT-2 inhibitor combination**

To confirm a heart failure therapeutic effect of the combination of the angiotensin II receptor blocker (ARB) and the sodium-glucose cotransporter-2 (SGLT-2) inhibitor of the present disclosure, it was confirmed whether the mRNA expression of the angiotensin converting enzyme (ACE) was inhibited.

Specifically, the experimental samples were prepared as shown in Table 3

[Table 3]

| Classification | Experimental sample | |
|---|---|---|
| | ARB | SGLT-2 inhibitior |
| Example 1 | Telmisartan 10 $\mu$M | Empagliflozin 5 $\mu$M |

(continued)

| Classification | Experimental sample | |
|---|---|---|
| | ARB | SGLT-2 inhibitior |
| Example 2 | Telmisartan 10 $\mu$M | Dapagliflozin 5 $\mu$M |
| Comparative Example 1 | Olmesartan 10 $\mu$M | Empagliflozin 5 $\mu$M |
| Comparative Example 2 | Olmesartan 10 $\mu$M | Dapagliflozin 5 $\mu$M |
| Comparative Example 3 | Telmisartan 10 $\mu$M | Canagliflozin 5 $\mu$M |
| Comparative Example 4 | Valsartan 10 $\mu$M | Empagliflozin 5 $\mu$M |
| Comparative Example 5 | - | Empagliflozin 2.5 $\mu$M |
| Comparative Example 6 | - | Empagliflozin 5 $\mu$M |
| Comparative Example 7 | - | Empagliflozin 10 $\mu$M |
| Comparative Example 8 | - | Empagliflozin 15 $\mu$M |
| Comparative Example 9 | - | Dapagliflozin 2.5 $\mu$M |
| Comparative Example 10 | - | Dapagliflozin 5 $\mu$M |
| Comparative Example 11 | - | Dapagliflozin 10 $\mu$M |
| Comparative Example 12 | - | Dapagliflozin 15 $\mu$M |
| Comparative Example 13 | Telmisartan 10 $\mu$M | - |
| Comparative Example 14 | Telmisartan 15 $\mu$M | - |
| Comparative Example 15 | - | Canagliflozin 5 $\mu$M |

In order to confirm the effect of each combination or single substance on the mRNA expression of ACE, the experimental samples in Table 3 were treated to H9c2 cells induced with LPS inflammation in Experimental Example 1-2, respectively, and then the mRNA expression of ACE was evaluated in the same manner as in Experimental Example 1-3.

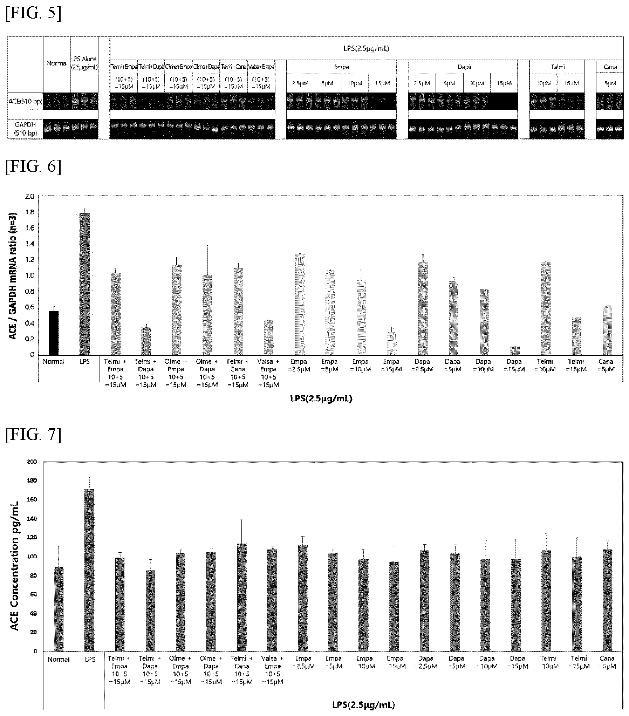

As a result, in the case of the Telmisartan, Empagliflozin, and Dapagliflozin-alone administration groups, the mRNA expression of ACE did not decrease to a normal level in the low-concentration administration groups of 2.5 $\mu$M to 10 $\mu$M, whereas in 15 $\mu$M high-concentration administration groups (Comparative Examples 8, 12, and 14), the mRNA expression of ACE was confirmed to decrease below the normal level. Meanwhile, among the combination administration groups, in Example 2, which was a group treated with 10 $\mu$M Telmisartan and 5 $\mu$M Dapagliflozin, it was confirmed that the mRNA expression of ACE was significantly inhibited compared to other combination administration groups (FIGS. 5 and 6).

Through these results, it was confirmed that the combination of Telmisartan and Dapagliflozin was the most effective ARB and SGLT-2 inhibitor combination for inhibiting the ACE expression.

**Experimental Example 3. Confirmation of angiotensin converting enzyme inhibitory activity according to protein expression inhibition of ARB and SGLT-2 inhibitor combination**

To confirm a heart failure therapeutic effect of the combination of the angiotensin II receptor blocker (ARB) and the sodium-glucose cotransporter-2 (SGLT-2) inhibitor of the present disclosure, it was confirmed whether the protein expression of the angiotensin converting enzyme (ACE) was inhibited.

Specifically, the H9c2 cells induced with LPS inflammation in Experimental Example 1-2 were treated with the experimental samples in Table 3 and the H9c2 cells were harvested to perform ELISA assay. Thereafter, the harvested cells were treated with 1 ml of an RIPA buffer and stirred at 4°C for 30 minutes to extract proteins. Thereafter, the proteins were centrifuged at 4°C and 10,000 rpm for 20 minutes, and the supernatant was transferred to a sterilized tube and stored at - 80°C for use in the ELISA assay. The ELISA assay was performed using the same method as Experimental Example 1-2.

As a result, among all single administration groups and combination administration groups, it was confirmed that the expression of ACE protein was significantly inhibited in Example 2, which was the group administered with 10 $\mu$M Telmisartan and 5 $\mu$M Dapagliflozin, and then the expression ACE protein was significantly inhibited in the group administered with 10 $\mu$M Telmisartan and 5 $\mu$M Empagliflozin (FIG. 7).

Through these results, it was confirmed that the combination of Telmisartan and Dapagliflozin or the combination of Telmisartan and Empagliflozin was the most effective combination of ARB and SGLT-2 inhibitor for inhibiting the ACE expression, and may be effectively applied to alleviate side effects caused by the use of a high-dose of ARB or SGLT-2 inhibitor.

**Experimental Example 4. Evaluation of toxicity of ARB and SGLT-2 inhibitor combination**

*4-1. Evaluation of single-dose toxicity*

The single-dose toxicity was evaluated for the combination of Telmisartan and Dapagliflozin, and the combination of Telmisartan and Empagliflozin, which were identified as the most effective combinations of ARBs and SGLT-2 inhibitors for inhibiting ACE expression.

Specifically, the groups consisted of groups administered with Dapagliflozin (Dapagliflozin propanediol hydrate) and Telmisartan combinations of 3.1/20 mg/kg/day (low-dose group), 6.2/40 mg/kg/day (medium-dose group), and 12.3/80 mg/kg/day (high-dose group), and singe-dose groups administered with Dapagliflozin 12.3 mg/kg/day and Telmisartan 80 mg/kg/day, respectively, groups administered with Empagliflozin and Telmisartan combinations of 6.25/20 mg/kg/day (low-dose group), 12.5/40 mg/kg/day (medium-dose group), and 25/80 mg/kg/day (high-dose group), and single-dose groups administered with Empagliflozin 25 mg/kg/day and Telmisartan 80 mg/kg/day, respectively, and a control group (0.5% MC aqueous solution). A single oral administration was performed to 5 male and 5 female 6-week-old Sprague-Dawley rats per group, and general symptoms were observed and body weights were measured for 15 days after administration, and after the end of the observation period, the rats were euthanized and autopsied.

As a result, no dead animals were observed in all of the administration groups during the observation period. In addition, as the results of the general symptom observation, weight measurement, and autopsy, no results were confirmed to be determined as the effects of the ARB and SGLT-2 inhibitor combination in all of the administration groups.

Through these results, it was confirmed that the approximate lethal doses according to a single oral administration of the Dapagliflozin (Dapagliflozin propanediol monohydrate) and Telmisartan combination, and the Empagliflozin and Telmisartan combination were more than 12.3/80 mg/kg and 25/80 mg/kg in both males and females, respectively.

*4-2. Evaluation of toxicity of 4-week repeated oral administration and determination of dose*

The dose was measured by evaluating toxicity of four repeated oral administration of the combination of Telmisartan and Dapagliflozin, and the combination of Telmisartan and Empagliflozin, which were identified as the most effective combinations of ARBs and SGLT-2 inhibitor for inhibiting ACE expression.

Specifically, groups were configured in the same manner as in Experimental Example 4-1, and the combinations were administered orally daily for 4 weeks to 5 male and 5 female 6-week-old Sprague-Dawley rats per group. During the test, general symptom observation, blood test, and body weight measurement were performed, and after the observation period ended, the rats were euthanized and autopsied.

As a result of body weight measurement, a tendency for inhibiting weight gain was observed in a high-dose group and a Telmisartan-alone administration group. Blood test results showed that BUN increased depending on a dose, GLU decreased in the Dapagliflozin-alone administration group and all high-dose groups, and RBC, HGB, and HCT decreased in all of the high-dose groups. It was confirmed that in all low, medium, and high-dose groups, the absolute and relative weights of the kidney were increased, and in all medium and high-dose groups, the absolute and relative weights of the heart were decreased. Meanwhile, lesions on the glandular stomach were identified in one male in the high-dose group and one female in the medium-dose group.

This trend was determined to be the excessive pharmaceutical action of Dapagliflozin and the class effect of ARB-based drugs. Therefore, the high dose of Dapagliflozin (Dapagliflozin propanediol monohydrate) and Telmisartan for the 13-week repeated-administration toxicity test was set to 9.2/60 mg/kg/day (45-fold), and the medium and low doses were set to 3.1/20 mg/kg/day and 1/6.7 mg/kg/day, respectively, by applying a dose ratio of 3.0.

**Experimental Example 5. Confirmation of optimal combination and formulation of angiotensin II receptor blocker and sodium glucose cotransporter-2 combinations**

*5-1. Confirmation of optimal combination of angiotensin II receptor blocker and sodium glucose cotransporter-2 combinations according to drug-drug compatibility test*

The optimal combination of an angiotensin II receptor blocker (ARB) and a sodium-glucose cotransporter-2 (SGLT-2) inhibitor that did not decompose and have high stability even after long-term storage was identified through a drug-drug compatibility test.

Specifically, after storing a mixture of ARB and SGLT-2 inhibitor for 4 weeks under accelerated stability test conditions (40 ± 2°C/75 ± 5% relative humidity), changes in the contents of each ingredient and flexible substance and changes in properties (color, agglomeration, etc.) were analyzed. Olmesartan, Valsartan, and Telmisartan were used as ARBs, and Empagliflozin, Dapagliflozin (Dapagliflozin propanediol monohydrate), and Canagliflozin were used as SGLT-2 inhibitors. The contents of each ingredient were configured as shown in Table 4.

[Table 4]

| Classification | Ingredient | |
|---|---|---|
| | ARB | SGLT-2 inhibitor |
| Example 3 | Telmisartan 40 mg | Empagliflozin 40 mg |
| Example 4 | Telmisartan 40 mg | Dapagliflozin 40 mg |
| Comparative Example 16 | Telmisartan 80 mg | - |
| Comparative Example 17 | - | Empagliflozin 80 mg |
| Comparative Example 18 | - | Dapagliflozin 80 mg |
| Comparative Example 19 | Olmesartan 80 mg | - |
| Comparative Example 20 | Valsartan 80 mg | - |
| Comparative Example 21 | - | Canagliflozin 80 mg |
| Comparative Example 22 | Telmisartan 40 mg | Canagliflozin 40 mg |
| Comparative Example 23 | Olmesartan 40 mg | Empagliflozin 40 mg |
| Comparative Example 24 | Olmesartan 40 mg | Dapagliflozin 40 mg |
| Comparative Example 25 | Valsartan 40 mg | Empagliflozin 40 mg |
| Comparative Example 26 | Valsartan 40 mg | Dapagliflozin 40 mg |

To analyze the contents of the main ingredients and the degree of production of flexible substances, 10T of tablets for each of mixtures of ARBs and SGLT-2 inhibitors stored for 1, 2, 3, and 4 weeks were placed in three 100 mL flasks, and added with a solution in which a 0.01 M hydrochloric acid aqueous solution and acetonitrile were mixed in an appropriate ratio to extract the main ingredients, stirred, and then analyzed by HPLC.

To observe the properties and viscosity of the main ingredients, 10 g of the mixtures of ARBs and SGLT-2 inhibitors stored for 1, 2, 3, and 4 weeks were taken and exposed to 100 ml of purified water at room temperature for 10 minutes, and then the properties and viscosity were identified.

As a result, in acceleration 3 week, it was confirmed that all indicators were unsuitable in Comparative Examples 22 to 26 among the mixtures of ARBs and SGLT-2 inhibitors, whereas the contents of the mixture of Telmisartan and Empagliflozin (Example 3) and the mixture of Telmisartan and Dapagliflozin (Example 4) were 98.6%/92.1% and 98.9%/99.0%, respectively, showing content changes of less than 10%, and the total amount of flexible substances was also found to be appropriate, and thus it was confirmed that the drug-drug combination was the most appropriate. However, from acceleration 3 week, the properties were found to be unsuitable, and thus it was confirmed that a formulation capable of maintaining the properties was required (Table 5).

[Table 5]

| Classification | Test item | Initial | Acceleration 1 week | Acceleration 2 week | Acceleration 3 week | Acceleration 4 week |
|---|---|---|---|---|---|---|
| Ex. 3 | content | 99.9/100 | 99.5/97.6 | 99.4/95.1 | 99.2/92.1 | 99/89.8 |
| | flexible substance | 0.1 or less | 1.0 or more | 1.0 or more | 1.0 or more | 5.0 or more |
| | property | suitable | suitable | suitable | unsuitable | unsuitable |
| Ex. 4 | content | 99.9/100.1 | 99.4/99.9 | 99.2/99.2 | 98.9/99 | 98.6/98.9 |
| | flexible substance | 0.1 or less | 0.1 or more | 0.1 or more | 1.0 or less | 1.0 or more |
| | property | suitable | suitable | suitable | unsuitable | unsuitable |

(continued)

| Classification | Test item | Initial | Acceleration 1 week | Acceleration 2 week | Acceleration 3 week | Acceleration 4 week |
|---|---|---|---|---|---|---|
| Com. Ex. 16 | content | 100.1 | 100.1 | 100 | 100 | 100 |
| | flexible substance | 0.1 or less | 0.1 or less | 0.1 or less | 0.1 or less | 0.1 or less |
| | property | suitable | suitable | suitable | suitable | suitable |
| Com. Ex. 17 | content | 99.9 | 97.3 | 95.1 | 85.4 | 80.2 |
| | flexible substance | 0.1 or less | 1.0 or less | 1.0 or more | 5.0 or more | 5.0 or more |
| | property | suitable | suitable | suitable | unsuitable | unsuitable |
| Com. Ex. 18 | content | 100.3 | 100.1 | 100.2 | 99.8 | 99 |
| | flexible substance | 0.1 or less | 0.1 or less | 0.1 or less | 0.1 or less | 1.0 or less |
| | property | suitable | suitable | suitable | unsuitable | unsuitable |
| Com. Ex. 19 | content | 99.9 | 99.7 | 99.7 | 99.5 | 99.4 |
| | flexible substance | 0.1 or less | 0.1 or more | 0.1 or more | 0.1 or more | 0.1 or more |
| | property | suitable | suitable | suitable | suitable | suitable |
| Com. Ex. 20 | content | 99.7 | 99.4 | 99.2 | 99 | 98.7 |
| | flexible substance | 0.1 or less | 0.1 or more | 0.1 or more | 0.1 or more | 0.1 or more |
| | property | suitable | suitable | suitable | suitable | suitable |
| Com. Ex. 21 | content | 99.5 | 95.2 | 89.2 | 85.2 | 80.1 |
| | flexible substance | 0.1 or less | 1.0 or less | 5.0 or more | 5.0 or more | 5.0 or more |
| | property | suitable | unsuitable | unsuitable | unsuitable | unsuitable |
| Com. Ex. 22 | content | 99.5/92.1 | 99.1/89.1 | 98.9/82.3 | 94.5/74.4 | 92.3/62.3 |
| | flexible substance | 0.1 or less | 5.0 or more | 5.0 or more | 5.0 or more | 5.0 or more |
| | property | suitable | unsuitable | unsuitable | unsuitable | unsuitable |
| Com. Ex. 23 | content | 100.1/99.9 | 99.1/90 | 95.4/84.6 | 92.3/72.8 | 90.1/60.5 |
| | flexible substance | 0.1 or less | 5.0 or more | 5.0 or more | 5.0 or more | 5.0 or more |
| | property | suitable | suitable | unsuitable | unsuitable | unsuitable |
| Com. Ex. 24 | content | 100.1/100 | 97.8/89.1 | 95/79.7 | 91.9/69.9 | 89.9/60 |
| | flexible substance | 0.1 or less | 5.0 or more | 5.0 or more | 5.0 or more | 5.0 or more |
| | property | suitable | suitable | unsuitable | unsuitable | unsuitable |
| Com. Ex. 25 | content | 100.2/99.9 | 95.5/95.4 | 93.3/90.1 | 90.8/85.5 | 90/80.7 |
| | flexible substance | 0.1 or less | suitable | 5.0 or more | 5.0 or more | 5.0 or more |
| | property | suitable | unsuitable | unsuitable | unsuitable | unsuitable |

(continued)

| Classification | Test item | Initial | Acceleration 1 week | Acceleration 2 week | Acceleration 3 week | Acceleration 4 week |
|---|---|---|---|---|---|---|
| Com. Ex. 26 | content | 100.3/99.7 | 95.5/95.7 | 91.1/92.3 | 89.1/87.9 | 87.1/81.1 |
| | flexible substance | 0.1 or less | 1.0 or more | 1.0 or more | 5.0 or more | 5.0 or more |
| | property | suitable | unsuitable | unsuitable | unsuitable | unsuitable |

*5-2. Preparation of bilayer tablet containing angiotensin II receptor blocker and sodium glucose cotransporter-2 combination by direct tableting process*

An ARB granule layer according to Table 6 was prepared through wet granulation, and the granules were prepared by drying after preparing in a semi-aqueous manner with the main ingredients, mannitol, meglumine, and sodium hydroxide as a solubilizing agent. All of the ingredients except for sodium stearyl fumarate and magnesium stearate as lubricants, among the compositions of the ARB layer and the SGLT-2 inhibitor layer, were sieved through a 20 mesh standard sieve, and then first pre-mixed with each layer composition, and mixed for at least 10 minutes using a 3D mixer (turbula mixer), respectively. Thereafter, the lubricant was added to each powder mixture and mixed again for 5 minutes. Finally, the mixture forming each layer was injected into each hopper of a two-layer tableting machine, applied with a compression force, and then coated with a coating material to prepare a bilayer tablet. Olmesartan, Valsartan, and Telmisartan were used as ARBs, and Empagliflozin, Dapagliflozin (Dapagliflozin propanediol monohydrate), and Canagliflozin were used as SGLT-2 inhibitors. The combination of each ingredient was configured as shown in Table 7.

[Table 6]

| Classification | Function | Ingredient | Parts by weight |
|---|---|---|---|
| ARB layer | API | ARB granules | 16.8 |
| | Excipient | Mannitol | 28.2 |
| | Lubricant | Sodium stearyl fumarate and magnesium stearate | 2.5 |
| SGLT-2 inhibitor layer | API | SGLT-2 inhibitor | 2.6 |
| | Excipient | Mannitol | 42.5 |
| | Disintegrant | Polyvinylpyrrolidone | 6.3 |
| | Lubricant | Magnesium stearate | 1.1 |
| Total | | | 100 |

[Table 7]

| Classification | Ingredient | |
|---|---|---|
| | ARB | SGLT-2 inhibitor |
| Example 5 | Telmisartan | Empagliflozin |
| Example 6 | Telmisartan | Dapagliflozin |
| Comparative Example 27 | Telmisartan | Canagliflozin |
| Comparative Example 28 | Valsartan | Empagliflozin |
| Comparative Example 29 | Valsartan | Dapagliflozin |
| Comparative Example 30 | Olmesartan | Empagliflozin |
| Comparative Example 31 | Olmesartan | Dapagliflozin |

*5-3. Preparation of active-coated tablet containing angiotensin II receptor blocker and sodium glucose cotransporter-2 combination by direct tableting process*

ARB granules according to Table 8 were prepared through wet granulation, and the granules were prepared by drying after preparing in a semi-aqueous manner with the main ingredients, mannitol, meglumine, and sodium hydroxide as a solubilizing agent. The ARB granules and respective ingredients were sieved through a 20-mesh standard sieve, first pre-mixed, and then mixed for at least 10 minutes using a 3D mixer (turbula mixer). Thereafter, the powder mixture was added with magnesium stearate as a lubricant, mixed again for 5 minutes, and the mixture was put into a hopper of a tableting machine, and then tablets were prepared using compression force. Finally, active-coated tablets were prepared by mixing the prepared tablets with an SGLT-2 inhibitor, Aerosil, and HPMC, and performing a second sub-coating with a coating substrate after first coating a main drug using an automatic coater, and the SGLT-2 inhibitor and ARB ingredients used were Dapagliflozin and Telmisartan, respectively.

[Table 8]

| Classification | Function | Ingredient | Parts by weight |
|---|---|---|---|
| ARB layer | API | ARB | 15.6 |
| | Excipient | Mannitol | 75.2 |
| | Lubricant | Sodium stearyl fumarate and Magnesium stearate | 2.5 |
| SGLT-2 inhibitor layer | API | SGLT-2 inhibitor | 2.4 |
| | Excipient | Aerosil | 0.4 |
| | Coating agent | HPMC | 3.9 |
| Total | | | 100 |

*5-4. Confirmation of optimal combination of angiotensin II receptor blocker and sodium glucose cotransporter-2 combination formulation according to preparation stability analysis*

An optimal combination of an angiotensin II receptor blocker (ARB) and sodium-glucose cotransporter-2 (SGLT-2) inhibitor combination formulation that did not decompose and have high stability even in long-term storage was identified by evaluating the preparation stability.

Specifically, the ARB and SGLT-2 inhibitor combined bilayer tablets prepared in Experimental Example 5-2 were stored for 6 months under accelerated stability test conditions ($40 \pm 2°C/75 \pm 5\%$ relative humidity) or long-term stability test conditions ($25 \pm 2°C/60 \pm 5\%$ relative humidity), and then changes in the contents of each ingredient and flexible substance, changes in properties (color, agglomeration, etc.), and changes in dissolution (interference, changes in dissolution, etc.) were analyzed.

The changes in the contents of each ingredient and flexible substance, and the changes in properties (color, agglomeration, etc.) were analyzed in the same manner as in Experimental Example 5-1, and the dissolution changes were analyzed using the method in Table 9 for the ARB and SGLT-2 inhibitor combined bilayer tablets and the active-coated tablets prepared in Experimental Examples 5-2 and 5-3.

[Table 9]

| Classification | | Content |
|---|---|---|
| Dissolution test condition | Dissolution method | Dissolution test method 2 of the Korean Pharmacopoeia (paddle method) |
| | Dissolution solution | pH 1.2 hydrochloric acid (HCl) solution |
| | Dissolution solution amount | 900 ml |
| | Dissolution device temperature | 37.5°C |
| | Paddle speed | 50 rpm |
| | Number of test groups | 4 |
| | Specimen collection time | 5, 10, 15, 30, 45, 60, 90, 120 minutes |
| Specimen analysis condition | Detector | UV spectrophotometer (measurement wavelength: 229 nm) |
| | Column | C18 (5 $\mu$m, 4.6 $\times$ 250 mm) |
| | Mobile phase | Using a solution in which 2.0 g of ammonium dihydrogen phosphate was weighed, dissolved in water to make exactly 1.0 L, and then added with 1 mol/L phosphoric acid, and mixed with 300 mL of a solution adjusted to pH 3.0 and 700 mL of methanol. |
| | Flow rate | 0.7 ml/min |
| | Column temperature | 35°C |

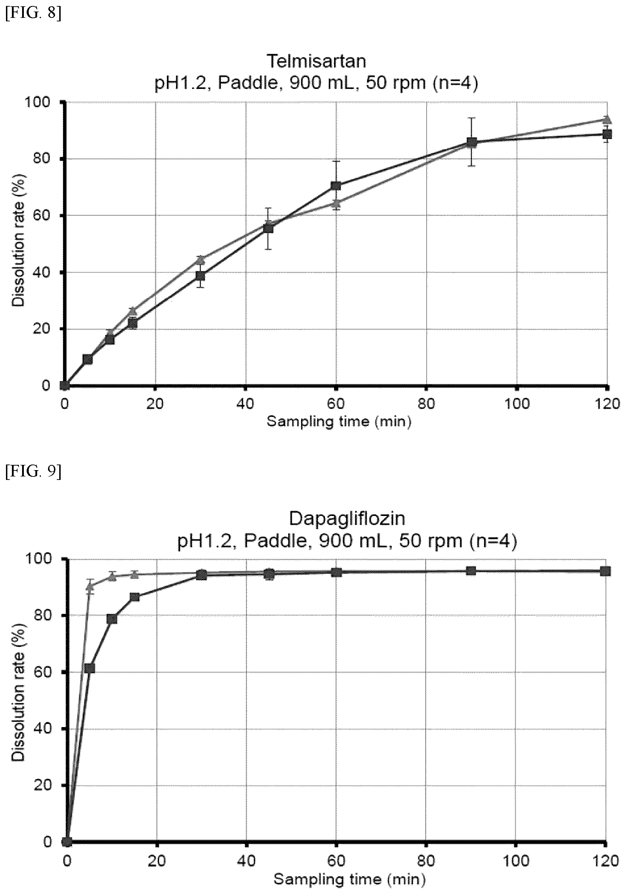

As a result, most of the indicators were found to be unsuitable in Comparative Examples 27 to 31 among the ARB and SGLT-2 inhibitor combined bilayer tablets at acceleration 6 month and long-term 6 month, whereas all of the indicators were found to be suitable in the Telmisartan and Empagliflozin combined bilayer tablets (Example 5) and the Telmisartan and Dapagliflozin (Dapagliflozin propanediol monohydrate) combined bilayer tablets (Example 6), which had excellent preparation stability (Table 10). In particular, it was confirmed that the properties that were unsuitable in the drug-drug compatibility test were also suitable after formulated as a combined bilayer tablet. Meanwhile, the dissolution patterns of the Telmisartan and Dapagliflozin formulations of Example 6 were confirmed as shown in FIGS. 8 and 9, respectively, and the dissolution patterns of the combined bilayer tablet and the active coating tablet were confirmed to be the same as each other.

[Table 10]

| Classification | Test item | Initial | Acceleration 1 month | Acceleration 3 month | Acceleration 6 month | Long-term 1 month | Long-term 3 month | Long-term 6 month |
|---|---|---|---|---|---|---|---|---|
| Ex. 5 | content | 100.3% | 100.1% | 100.1% | 99.7% | 100.3% | 99.9% | 100.1% |
| | flexible substance | Total 0.1% or less | Total 0.1% or less | Total 0.15% or less | Total 0.2% or less | Total 0.1% or less | Total 0.1% or less | Total 0.1% or less |
| | property | suitable | suitable | suitable | suitable | suitable | suitable | suitable |
| | Dissolution | suitable | suitable | suitable | suitable | suitable | suitable | suitable |
| Ex. 6 | content | 100.1% | 99.9% | 100.0% | 99.8% | 100.1% | 100.1% | 100.2% |
| | flexible substance | Total 0.1% or less | Total 0.1% or less | Total 0.1% or less | Total 0.2% or less | Total 0.1% or less | Total 0.1% or less | Total 0.1% or less |
| | property | suitable | suitable | suitable | suitable | suitable | suitable | suitable |
| | Dissolution | suitable | suitable | suitable | suitable | suitable | suitable | suitable |
| Com. Ex. 27 | content | 99.8% | 95.0% | 90.1% | 82.5% | 99.0% | 96.9% | 94.3% |
| | flexible substance | Total 0.1% or less | Total 1.0% or less | Total 3.0% or less | Total 5% or more | Total 0.1% or less | Total 1.0% or less | Total 1.0% or less |
| | property | suitable | unsuitable | unsuitable | unsuitable | unsuitable | unsuitable | unsuitable |
| | Dissolution | suitable | unsuitable | unsuitable | unsuitable | unsuitable | unsuitable | unsuitable |
| Com. Ex. 28 | content | 99.9% | 97.4% | 89.7% | 80.4% | 99.2% | 95.4% | 95.0% |
| | flexible substance | Total 0.1% or less | Total 1.0% or less | Total 5% or more | Total 5% or more | Total 0.1% or less | Total 1.0% or less | Total 3.0% or less |
| | property | suitable | suitable | unsuitable | unsuitable | unsuitable | unsuitable | unsuitable |
| | Dissolution | suitable | unsuitable | unsuitable | unsuitable | unsuitable | unsuitable | unsuitable |
| Com. Ex. 29 | content | 100.2% | 100.1% | 87.7% | 79.5% | 98.6% | 94.8% | 95.3% |
| | flexible substance | Total 0.1% or less | Total 0.1% or less | Total 5% or more | Total 5% or more | Total 0.1% or less | Total 1.0% or less | Total 3.0% or less |
| | property | suitable | suitable | unsuitable | unsuitable | unsuitable | unsuitable | unsuitable |
| | Dissolution | suitable | unsuitable | unsuitable | unsuitable | unsuitable | unsuitable | unsuitable |

(continued)

| Classification | Test item | Initial | Acceleration 1 month | Acceleration 3 month | Acceleration 6 month | Long-term 1 month | Long-term 3 month | Long-term 6 month |
|---|---|---|---|---|---|---|---|---|
| Com. Ex. 30 | content | 100.1% | 98.7% | 97.7% | 95.8% | 99.5% | 98.5% | 97.3% |
| | flexible substance | Total 0.1% or less | Total 0.1% or less | Total 1.0% or less | Total 3.0% or less | Total 0.1% or less | Total 1.0% or less | Total 1.0% or more |
| | property | suitable | suitable | suitable | unsuitable | unsuitable | unsuitable | unsuitable |
| | Dissolution | suitable | suitable | unsuitable | unsuitable | unsuitable | unsuitable | unsuitable |
| Com. Ex. 31 | content | 99.9% | 98.9% | 98.1% | 94.3% | 99.6% | 98.9% | 97.9% |
| | flexible substance | Total 0.1% or less | Total 0.1% or less | Total 1.0% or less | Total 3.0% or less | Total 0.1% or less | Total 1.0% or less | Total 1.0% or more |
| | property | suitable | suitable | suitable | unsuitable | unsuitable | unsuitable | unsuitable |
| | Dissolution | suitable | suitable | unsuitable | unsuitable | unsuitable | unsuitable | unsuitable |

**Experimental Example 6. Confirmation of pharmacokinetics of single oral administration of combined bilayer tablet of Dapagliflozin and Telmisartan**

To identify the pharmacokinetics of the combined bilayer tablet of Dapagliflozin and Telmisartan, a single oral administration pharmacokinetic test was performed using beagle dogs.

Specifically, total 24 male beagle dogs (6 per group) were administered with 10 mg of Forxiga Tab. (Dapagliflozin, control drug 1) and 80 mg of Micardis Tab. (Telmisartan, control drug 2) as a control drug alone or in combination, and the combined bilayer tablet of Example 6 (80 mg of Telmisartan and 10 mg of Dapagliflozin (12.3 mg as Dapagliflozin propanediol hydrate)) as a test drug. The control and test drugs were administered orally once by a single tablet, and were crossed over (4 × 4 cross over) four times at one-week intervals. Fasting was performed from 5 to 6 pm the day before administration. The control and test drugs were administered into the tongue root, and then administered with approximately 15 ml of water, and feed was provided approximately 4 hours after administration. Blood was collected at 0 (blank), 0.25, 0.5, 0.75, 1, 1.5, 2, 3, 4, 5, 6, 8, 12, and 24 hours (14 times) after administration of the control and test drugs, and left by a 7-day drug-free period. The AUC, $C_{max}$, $T_{max}$, $t_{1/2}$, etc. of Dapagliflozin, Telmisartan, and the combined bilayer tablet of Example 6 were evaluated.

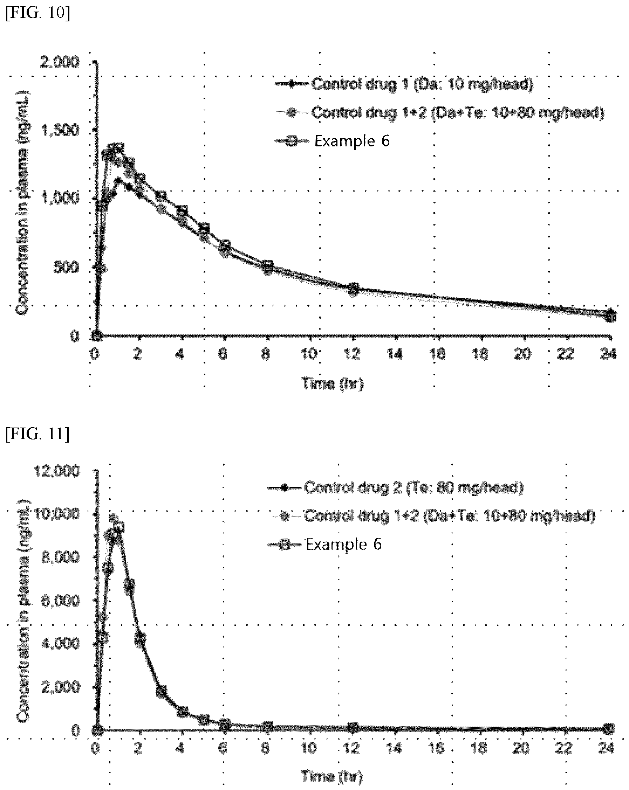

As a result, in the case of Dapagliflozin, a difference in $AUC_{all}$ between groups in the single administration group and the combined administration group was 1.0 times, and thus it was confirmed that there was no drug-drug interaction (DDI) at all, and the equivalence in the confidence interval was confirmed to be equivalent by satisfying the judgment criterion (log 0.8 to 1.25) (Table 11 and FIG. 10). In the case of Telmisartan, a difference in $AUC_{all}$ between groups in the single administration group and the combined administration group was 1.0 times, and thus it was confirmed that there was no drug-drug interaction (DDI), and a difference in log-transformed average was equivalent as $AUC_{all}$ 1.01 and $C_{max}$ 0.98 by satisfying the judgment criteria (Table 12 and FIG. 11).

[Table 11]

| Drug/Dose (mg/head) | Classification | $AUC_{all}$ (ng*hr/ml) | $C_{max}$ (ng/ml) | $T_{max}$ (hr) | t1/2 (hr) |
|---|---|---|---|---|---|
| Control drug 1 | Mean | 10,041 | 1,433 | 0.83 | 7.78 |
| | S,D, | 958 | 217 | 0.63 | 0.84 |
| | n | 24 | 24 | 24 | 24 |
| Control drug 1 + Control drug 2 | Mean | 10,495 | 1,450 | 0.88 | 8.63 |
| | S,D, | 928 | 250 | 0.35 | 1.57 |
| | n | 24 | 24 | 24 | 24 |
| Example 6 | Mean | 11,563 | 1,631 | 0.77 | 8.51 |
| | S,D, | 1,110 | 239 | 0.47 | 0.99 |
| | n | 24 | 24 | 24 | 24 |

[Table 12]

| Drug/Dose (mg/head) | Classification | $AUC_{all}$ (ng*hr/ml) | $C_{max}$ (ng/ml) | $T_{max}$ (hr) | t1/2 (hr) |
|---|---|---|---|---|---|
| Control drug 2 | Mean | 20,063 | 10,373 | 0.77 | 7.92 |
| | S,D, | 4,645 | 2,861 | 0.37 | 10.02 |
| | n | 24 | 24 | 24 | 24 |
| Control drug 1 + Control drug 2 | Mean | 20,327 | 10,983 | 0.74 | 6.92 |
| | S,D, | 5,491 | 3,961 | 0.32 | 7.29 |
| | n | 24 | 24 | 24 | 24 |
| Example 6 | Mean | 20,444 | 10,721 | 0.82 | 7.11 |
| | S,D, | 2,742 | 2,210 | 0.35 | 7.40 |
| | n | 24 | 24 | 24 | 24 |

**Experimental Example 7. Confirmation of body weight loss and heart weight lose effects of ARB and SGLT-2 inhibitor combination**

The body weight loss and heart weight lose effects were evaluated for the combination of Telmisartan and Dapagliflozin, which was identified as the most effective combination of ARB and SGLT-2 inhibitor for inhibiting ACE expression.

Specifically, the groups consisted of groups administered with Dapagliflozin and Telmisartan combinations of 1/8 mg/kg/day (low-dose group, G2), 3/24 mg/kg/day (medium-dose group, G3), and 9/72 mg/kg/day (high-dose group, G4), groups administered with Dapagliflozin 9 mg/kg/day (G5) and Telmisartan 72 mg/kg/day (G6) alone, and a control group (0.5% MC aqueous solution, G1). The dose volume for each group was diluted with a 0.5% MC aqueous solution and set to 10 mL/kg. 10 male and 10 female six-week-old Sprague-Dawley rats per group were orally administered repeatedly for 13 weeks, and after administration, general symptoms were observed and body weights were measured, and after the observation period was completed, the rats were euthanized and autopsied, and then the heart weights were measured.

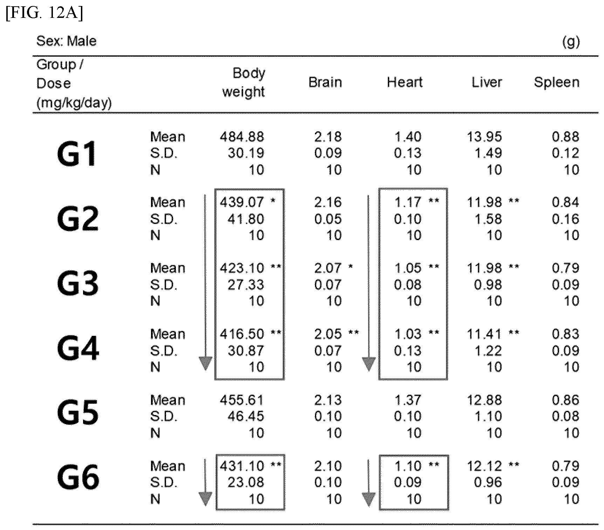

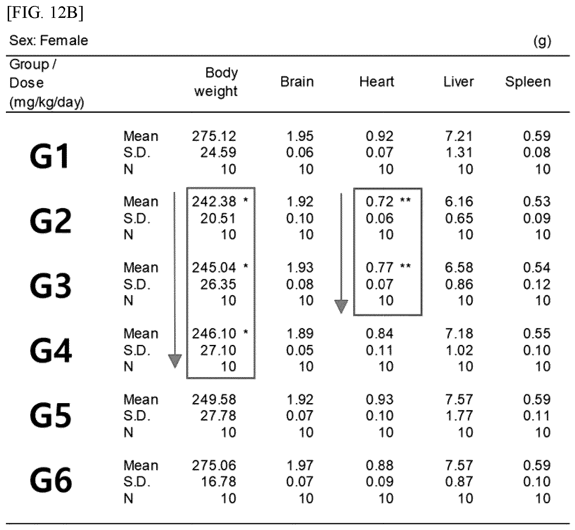

As a result, significant body weight loss and heart weight loss were confirmed in the low-dose, medium-dose, and high-dose groups of the Dapagliflozin and Telmisartan combination, and in particular, significant body weight loss and heart weight loss were confirmed in the low-dose and medium-dose groups of the Dapagliflozin and Telmisartan combination in female rats (FIGS. 12A and 12B).

Through these results, it was confirmed that cardiac hypertrophy associated with heart failure could be reduced by the Dapagliflozin and Telmisartan combination.

**Experimental Example 8. Summary of Phase 1 clinical trial (Clinical Research Information Service/CRIS: PRE20230908-003, Clinicaltrials.gov/NCT06063109)**

_8-1. Research title_

2 intervention group, published, single-sequence, repeated oral administration crossover design clinical trials to evaluate safety and pharmacokinetic interactions when administering THP-00101 and THP-00102 in healthy adult volunteers

_8-2. Research purpose_

The research purpose was to explore the interaction between two clinical trial drugs by comparing and analyzing the pharmacokinetics and safety at a steady state during repeated oral administration of THP-00101 (Telmisartan 80 mg) and THP-00102 (Dapagliflozin 10 mg (12.3 mg as Dapagliflozin propanediol hydrate)) alone or in combination in healthy adult volunteers.

_8-3. Research type_

An interventional research test was conducted by 2 intervention group, published, single-sequence, and repeated oral administration crossover design.

_8-4. Number of subjects_

50 people

_8-5. Result_

As a result of the drug interaction test of the main ingredients of the drug, Telmisartan 80 mg (THP-00102) and Dapagliflozin 10 mg (THP-00101), it was confirmed that in the presence of Dapagliflozin, the $C_{max}$ of Telmisartan was approximately 1.19 times (T/R ratio 1.19, 90% confidence interval 0.99 to 1.43) and the AUC was approximately 1.09 times (T/R ratio 1.09, 90% confidence interval 1.01 to 1.18). In addition, it was confirmed that in the presence of Telmisartan, the $C_{max}$ of Dapagliflozin was approximately 1.02 times (T/R ratio 1.02, 90% confidence interval 0.93 to 1.12) and the AUC was approximately 1.00 times (T/R ratio 1.00, 90% confidence interval 0.97 to 1.03).

The present disclosure has been described above with reference to the examples thereof. It will be understood to those skilled in the art that the present disclosure may be implemented as modified forms without departing from an essential characteristic of the present disclosure. Therefore, the disclosed examples should be considered in an illustrative viewpoint rather than a restrictive viewpoint. The scope of the present disclosure is defined by the appended claims rather than by the foregoing description, and all differences within the scope of equivalents thereof should be construed as being included in the present disclosure.

**Claims**

1. An oral pharmaceutical preparation comprising:

   Telmisartan or a pharmaceutically acceptable salt thereof as a first pharmaceutical ingredient;
   a sodium-glucose cotransporter-2 (SGLT-2) inhibitor or a pharmaceutically acceptable salt thereof as a second pharmaceutical ingredient; and
   an excipient.

2. The oral pharmaceutical preparation of claim 1, wherein the SGLT-2 inhibitor is Dapagliflozin or Empagliflozin.

3. The oral pharmaceutical preparation of claim 1, wherein the weight ratio of the first pharmaceutical ingredient and the second pharmaceutical ingredient is 4 : 1 to 8 : 1.

4. The oral pharmaceutical preparation of claim 1, wherein the weight ratio of the entire pharmaceutical ingredient and the excipient is 1 : 2 to 1 : 5.

5. The oral pharmaceutical preparation of claim 1, wherein the preparation is a tablet.

6. The oral pharmaceutical preparation of claim 5, wherein the tablet comprises

   40 to 80 mg of the first pharmaceutical ingredient; and
   10 mg of the second pharmaceutical ingredient.

7. The oral pharmaceutical preparation of claim 5, wherein when the tablet is subjected to a dissolution test at a paddle rotation rate of 50 rpm in a pH 1.2 hydrochloric acid solution according to a dissolution test method 2 of the Korean Pharmacopoeia,

   the dissolution rate of the first pharmaceutical ingredient is 40% or more for 30 minutes and 85% or more for 90 minutes, and
   the dissolution rate of the second pharmaceutical ingredient is 60% or more for 5 minutes and 95% or more for 30 minutes.

8. A pharmaceutical composition for preventing or treating type 2 diabetes, hypertension, hypertension with diabetes, or heart failure, comprising the oral pharmaceutical preparation of claim 1.

9. A method for preventing or treating type 2 diabetes, hypertension, hypertension with diabetes, or heart failure, comprising administering the oral pharmaceutical preparation of claim 1 to a subject.

[FIG. 1A]

[FIG. 1B]

[FIG. 2A]

24h Treatment

[FIG. 2B]

48h Treatment

[FIG. 3]

[FIG. 4]

Lane 1 : Normal    Lane 3 : Olmesartan (10μM)    Lane 5 : Telmisartan (10μM)
Lane 2 : LPS    Lane 4 : Olmesartan (50μM)    Lane 6 : Telmisartan (25μM)

[FIG. 5]

[FIG. 6]

[FIG. 7]

[FIG. 8]

Telmisartan
pH1.2, Paddle, 900 mL, 50 rpm (n=4)

[FIG. 9]

Dapagliflozin
pH1.2, Paddle, 900 mL, 50 rpm (n=4)

[FIG. 10]

[FIG. 11]

[FIG. 12A]

Sex: Male          (g)

| Group / Dose (mg/kg/day) | | Body weight | Brain | Heart | Liver | Spleen |
|---|---|---|---|---|---|---|
| G1 | Mean | 484.88 | 2.18 | 1.40 | 13.95 | 0.88 |
| | S.D. | 30.19 | 0.09 | 0.13 | 1.49 | 0.12 |
| | N | 10 | 10 | 10 | 10 | 10 |
| G2 | Mean | 439.07 * | 2.16 | 1.17 ** | 11.98 ** | 0.84 |
| | S.D. | 41.80 | 0.05 | 0.10 | 1.58 | 0.16 |
| | N | 10 | 10 | 10 | 10 | 10 |
| G3 | Mean | 423.10 ** | 2.07 * | 1.05 ** | 11.98 ** | 0.79 |
| | S.D. | 27.33 | 0.07 | 0.08 | 0.98 | 0.09 |
| | N | 10 | 10 | 10 | 10 | 10 |
| G4 | Mean | 416.50 ** | 2.05 ** | 1.03 ** | 11.41 ** | 0.83 |
| | S.D. | 30.87 | 0.07 | 0.13 | 1.22 | 0.09 |
| | N | 10 | 10 | 10 | 10 | 10 |
| G5 | Mean | 455.61 | 2.13 | 1.37 | 12.88 | 0.86 |
| | S.D. | 46.45 | 0.10 | 0.10 | 1.10 | 0.08 |
| | N | 10 | 10 | 10 | 10 | 10 |
| G6 | Mean | 431.10 ** | 2.10 | 1.10 ** | 12.12 ** | 0.79 |
| | S.D. | 23.08 | 0.10 | 0.09 | 0.96 | 0.09 |
| | N | 10 | 10 | 10 | 10 | 10 |

[FIG. 12B]

Sex: Female                              (g)

| Group / Dose (mg/kg/day) | | Body weight | Brain | Heart | Liver | Spleen |
|---|---|---|---|---|---|---|
| G1 | Mean | 275.12 | 1.95 | 0.92 | 7.21 | 0.59 |
|  | S.D. | 24.59 | 0.06 | 0.07 | 1.31 | 0.08 |
|  | N | 10 | 10 | 10 | 10 | 10 |
| G2 | Mean | 242.38 * | 1.92 | 0.72 ** | 6.16 | 0.53 |
|  | S.D. | 20.51 | 0.10 | 0.06 | 0.65 | 0.09 |
|  | N | 10 | 10 | 10 | 10 | 10 |
| G3 | Mean | 245.04 * | 1.93 | 0.77 ** | 6.58 | 0.54 |
|  | S.D. | 26.35 | 0.08 | 0.07 | 0.86 | 0.12 |
|  | N | 10 | 10 | 10 | 10 | 10 |
| G4 | Mean | 246.10 * | 1.89 | 0.84 | 7.18 | 0.55 |
|  | S.D. | 27.10 | 0.05 | 0.11 | 1.02 | 0.10 |
|  | N | 10 | 10 | 10 | 10 | 10 |
| G5 | Mean | 249.58 | 1.92 | 0.93 | 7.57 | 0.59 |
|  | S.D. | 27.78 | 0.07 | 0.10 | 1.77 | 0.11 |
|  | N | 10 | 10 | 10 | 10 | 10 |
| G6 | Mean | 275.06 | 1.97 | 0.88 | 7.57 | 0.59 |
|  | S.D. | 16.78 | 0.07 | 0.09 | 0.87 | 0.10 |
|  | N | 10 | 10 | 10 | 10 | 10 |

## INTERNATIONAL SEARCH REPORT

| International application No. |
|---|
| **PCT/KR2023/019185** |

| A. | CLASSIFICATION OF SUBJECT MATTER |
|---|---|

**A61K 9/20**(2006.01)i; **A61K 31/4184**(2006.01)i; **A61K 31/7034**(2006.01)i; **A61K 45/06**(2006.01)i; **A61P 3/10**(2006.01)i; **A61P 9/12**(2006.01)i; **A61P 9/04**(2006.01)i

According to International Patent Classification (IPC) or to both national classification and IPC

| B. | FIELDS SEARCHED |
|---|---|

Minimum documentation searched (classification system followed by classification symbols)

A61K 9/20(2006.01); A61K 31/155(2006.01); A61K 31/41(2006.01); A61K 31/4178(2006.01); A61K 31/506(2006.01); A61K 31/70(2006.01); A61K 31/7034(2006.01); A61K 41/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 안지오텐신 II 수용체 차단제(angiotensin II receptor blocker, ARB), 텔미사르탄(telmisartan), 나트륨 포도당 운반체-2 저해제(sodium-glucose cotransporter-2 inhibitor, SGLT-2 inhibitor), 다파글리플로진(dapagliflozin), 엠파글리플로진(empagliflozin), 당뇨병(diabetes mellitus), 고혈압(hypertension), 심부전(cardiac failure)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-1943382 B1 (AUTOTELIC BIO INC.) 29 January 2019 (2019-01-29)<br>See abstract; claims 1 and 5-7; and paragraphs [0004], [0015], [0018], [0023]-[0026], [0043], [0054], [0059], [0108], [0113] and [0118]. | 1-8 |
| X | KR 10-2131359 B1 (AUTOTELIC BIO INC. et al.) 07 July 2020 (2020-07-07)<br>See claims 1-7; and paragraphs [0038], [0049] and [0051]-[0053]. | 1-8 |
| A | KR 10-2020-0094187 A (IDORSIA PHARMACEUTICALS LTD.) 06 August 2020 (2020-08-06)<br>See entire document. | 1-8 |
| A | KR 10-2021-0027229 A (ASTRAZENECA AB) 10 March 2021 (2021-03-10)<br>See entire document. | 1-8 |
| A | WO 2021-207723 A2 (CHINOOK THERAPEUTICS, INC. et al.) 14 October 2021 (2021-10-14)<br>See entire document. | 1-8 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **04 March 2024** | **04 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

<table>
<tr><td>**INTERNATIONAL SEARCH REPORT**</td><td>International application No.<br>**PCT/KR2023/019185**</td></tr>
</table>

| **Box No. I** | **Nucleotide and/or amino acid sequence(s) (Continuation of item 1.c of the first sheet)** |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/KR2023/019185** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **9**
   because they relate to subject matter not required to be searched by this Authority, namely:

   Claim 9 pertains to a method for treatment of the human body,
   and thus pertains to a subject matter on which the International Searching Authority is not required to carry
   out an international search under the provisions of PCT Article 17(2)(a)(i) and PCT Rule 39.1(iv).

2. ☐ Claims Nos.:
   because they relate to parts of the international application that do not comply with the prescribed requirements to such an
   extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

# EP 4 640 213 A1

## INTERNATIONAL SEARCH REPORT
### Information on patent family members

International application No.

**PCT/KR2023/019185**

| Patent document cited in search report | | | Publication date (day/month/year) | Patent family member(s) | | | Publication date (day/month/year) |
|---|---|---|---|---|---|---|---|
| KR | 10-1943382 | B1 | 29 January 2019 | CN | 110520133 | A | 29 November 2019 |
| | | | | JP | 2020-534347 | A | 26 November 2020 |
| | | | | JP | 6993502 | B2 | 13 January 2022 |
| | | | | US | 10980822 | B2 | 20 April 2021 |
| | | | | US | 2020-0054656 | A1 | 20 February 2020 |
| | | | | WO | 2019-059557 | A2 | 28 March 2019 |
| KR | 10-2131359 | B1 | 07 July 2020 | KR | 10-2020-0028796 | A | 17 March 2020 |
| | | | | WO | 2020-050677 | A1 | 12 March 2020 |
| KR | 10-2020-0094187 | A | 06 August 2020 | CN | 111405899 | A | 10 July 2020 |
| | | | | EP | 3716979 | A1 | 07 October 2020 |
| | | | | JP | 2021-504423 | A | 15 February 2021 |
| | | | | JP | 7404235 | B2 | 25 December 2023 |
| | | | | US | 2021-0169881 | A1 | 10 June 2021 |
| | | | | WO | 2019-106066 | A1 | 06 June 2019 |
| KR | 10-2021-0027229 | A | 10 March 2021 | CN | 112752582 | A | 04 May 2021 |
| | | | | EP | 4021507 | A1 | 06 July 2022 |
| | | | | JP | 2022-530575 | A | 30 June 2022 |
| | | | | JP | 2023-025207 | A | 21 February 2023 |
| | | | | KR | 10-2022-0044234 | A | 07 April 2022 |
| | | | | KR | 10-2359799 | B1 | 09 February 2022 |
| | | | | US | 10973836 | B2 | 13 April 2021 |
| | | | | US | 2021-0060043 | A1 | 04 March 2021 |
| | | | | US | 2021-0260083 | A1 | 26 August 2021 |
| | | | | WO | 2021-037400 | A1 | 04 March 2021 |
| WO | 2021-207723 | A2 | 14 October 2021 | CN | 115768421 | A | 07 March 2023 |
| | | | | EP | 4132509 | A2 | 15 February 2023 |
| | | | | JP | 2023-521169 | A | 23 May 2023 |
| | | | | KR | 10-2023-0015899 | A | 31 January 2023 |
| | | | | US | 2023-0270718 | A1 | 31 August 2023 |
| | | | | WO | 2021-207723 | A3 | 16 December 2021 |

Form PCT/ISA/210 (patent family annex) (July 2022)

34